# EUROPEAN PATENT SPECIFICATION

(11) **EP 4 211 231 B1**
(45) Date of publication and mention of the grant of the patent: **26.06.2024**
(21) Application number: 21777271.4
(22) Date of filing: 10.09.2021
(51) Int. Cl.: C12N 7/00, A61K 48/00, C12N 15/864, C07K 14/015, C07K 14/005, C12N 15/86

(54) **PEPTIDE-MODIFIED AAV CAPSID**
PEPTIDMODIFIZIERTES AAV-KAPSID
CAPSIDES AAV MODIFIÉES PAR PEPTIDE

(30) Priority: 10.09.2020 EP 20306005
(43) Date of publication of application: 19.07.2023
(73) Proprietor: GENETHON, 91000 Evry-Courcouronnes (FR); Institut National de la Santé et de la Recherche Médicale (INSERM), 75013 Paris (FR); Universite d'Evry Val d'Essonne, 91000 Evry (FR)
(72) Inventor: RICHARD, Isabelle, 91100 CORBEIL ESSONNES (FR); DOMINGUEZ, Natalia, 38004 Santa Cruz de Tenerife (ES)
(74) Representative: Plasseraud IP
(86) International application number: PCT/EP2021/074964
(87) International publication number: WO 2022/053630

(56) References cited:
- WO-A1-2015/006743
- WO-A1-2019/207132
- WO-A2-2018/022608
- STEFAN MICHELFELDER ET AL: "Successful Expansion but Not Complete Restriction of Tropism of Adeno-Associated Virus by In Vivo Biopanning of Random Virus Display Peptide Libraries", PLOS ONE, vol. 4, no. 4, 9 April 2009 (2009-04-09), page e5122, XP055147203, DOI: 10.1371/journal.pone.0005122

## Description

### FIELD OF THE INVENTION

The invention relates to a peptide-modified AAV capsid with improved tropism, in particular increased muscle tropism and/or reduced liver tropism as defined in the claims. The invention relates also to the derived recombinant AAV vector particle packaging a gene of interest, and its use in gene therapy, in particular for treating muscle diseases.

### BACKGROUND OF THE INVENTION

Recombinant Adeno-Associated Virus (rAAV) vectors are widely used for *in vivo* gene transfer and clinical trials using AAV vectors are currently taking place for the treatment of a number of diseases.

AAV is a non-pathogenic virus belonging to the genus Dependoparvovirus within the family Parvoviridae. AAV is a non-enveloped virus composed of a capsid of about 26 nm in diameter and a single-stranded DNA genome of 4.7 kb. The genome carries two genes, rep and cap, flanked by two palindromic regions named Inverted terminal Repeats (ITR) that serve as the viral origins of replication and the packaging signal. The cap gene codes for three structural proteins VP1, VP2 and VP3 that compose the icosahedral AAV capsid through alternative splicing and translation from different start codons. VP1, VP2 and VP3 share the same C-terminal end which is all of VP3. Using AAV2 has a reference, VP1 has a 735 amino acid sequence (GenBank accession number YP_680426.1 accessed on 13 August 2018); VP2 (598 amino acids) starts at the Threonine 138 (T138) and VP3 (533 amino acids) starts at the methionine 203 (M203). The three different VPs contribute in a 1 VP1: 1 VP2: 10 VP3 ratio to the AAV2 capsid. The capsids of all AAV serotypes are assembled from 60 VP monomers with approximately 50 copies of VP3, 5 copies of VP 2 and 5 copies of VP1. The rep gene encodes four proteins required for viral replication Rep78, Rep68, Rep52 and Rep40. Recombinant AAV vectors encapsidate an ITR-flanked rAAV genome in which a therapeutic gene expression cassette replaces the AAV protein coding-sequences.

Tissue specificity is determined by the capsid serotype and commonly used AAV serotypes isolated from human and non-human primates can transduce specific organs more efficiently than others, such as AAV6, AAV8, AAV9 and AAV-rh74 in muscle tissue.

However, all commonly used naturally occurring AAV serotypes have a propensity to accumulate within the liver. This causes problems, in particular when the AAV vector is administered by the systemic route. Firstly, a transgene aimed to be expressed in muscle may have toxic effects on the liver. Secondly, AAV vector entry in liver reduces the amount of vector available for muscle or nervous tissue. Consequently, higher doses of AAV vectors are required. This increases the possibility to induce liver toxicity and the cost of vector production.

WO2019/193119 discloses a hybrid AAV9.rh74 capsid which advantageously combines a drastically reduced liver tropism compared to both AAV9 and AAVrh74 parent serotypes and high level gene transduction efficiencies in skeletal and cardiac muscles.

Libraries of AAV capsid variants displaying short random peptides on the surface of various AAV serotypes have been generated to screen for gene therapy vectors with altered cell specificities and/or transduction efficiencies (Börner et al., Molecular Therapy, April 2020, 28, 1017-1032; Kienle EC (Dissertation for the degree of Doctor of natural Sciences, Combined Faculties for the Natural Sciences and for Mathematics of the Ruperto-Carola University of Heidelberg, Germany, 2014; WO 2019/207132; Michelfelder et al., PLoS ONE, 2009, 4, e5122; Review in Büning et al., Molecular Therapy: Methods & Clinical Development, 2019, 12, 248-).

A capsid-modified AAV2 vector displaying the peptide RGDLGLS (SEQ ID NO: 1 or P1) selected *in vitro* on primary breast tumor cells did not transduce the primary breast tumors *in vivo* (Michelfelder et al., precited). P1 in conjunction with AAV1, AAV2, AAV7 to 9 and AAVrh10 improved neuronal cell transduction *in vitro* (Kienle EC, precited). It has been reported that AAV9 vector displaying peptide P1 performs well in human astrocytes cultures and has an enhanced muscle tropism as well as detargeting from the liver (Börner *et al.,* precited; WO 2019/207132).

In all of these references, the cell-targeting peptide insertion strategy involves modifications of the AAV capsid sequence upstream and downstream of the insertion site. Said insertion strategies had the aim to facilitate cloning of libraries AAV capsid variants. The impact of the number and nature of amino acids flanking the cell-targeting peptide inserted in the AAV capsid on vector transduction efficiency *in vitro* was shown to be unpredictable because peptide-specific (Börner *et al.,* precited).

The ability to transduce muscle efficiently, selectively and safely with systemically-delivered AAV vectors would be beneficial for gene therapy of many human diseases.

### SUMMARY OF THE INVENTION

The inventors have surprisingly shown that the insertion of peptide P1 in AAV capsid without modifying the capsid sequence upstream and downstream of the insertion site can further improve muscle tropism and liver detargeting.

Therefore, the invention relates to a modified adeno-associated virus (AAV) capsid protein comprising a muscle-targeting peptide insertion between two consecutive amino acids of the capsid protein sequence and without any modification of the capsid protein amino acid sequence upstream and downstream of the insertion site, wherein the targeting peptide comprises the sequence SEQ ID NO: 1 and a sequence of small amino-acids at the N-terminal and C-terminal end(s) of SEQ ID NO: 1, and wherein the insertion site is into the variable region IV, V or VIII of the AAV capsid protein sequence.

In some embodiments, the sequence of small amino-acids consists of up to five amino acids selected from A, G, N, T, D, L and S; preferably G, GST, AAA, SGS, SGA, AGA, GAA or ALA at the N-terminal end and G, SG, AA, TG, GG, SA, AG or GA at the C-terminal end; more preferably AAA at the N-terminal end and SG at the C-terminal end of SEQ ID NO: 1.

In some embodiments, the muscle-targeting peptide consists of a sequence from 9 amino acids to up to 25, 20 or 15 amino acids.

In some embodiments, the insertion is at a position selected from positions 587, 588, 589, 453, 520, 584 and 585, according to the numbering in AAV2 capsid protein sequence.

In some embodiments, the modified AAV capsid protein is from an AAV serotype selected from the group consisting of: AAV6, AAV8, AAV9, AAVrh74 and AAV9.rh74; preferably AAV9 or AAV9.rh74.

In some preferred embodiments, the modified AAV capsid protein is from AAV9.rh74 and comprises the muscle-targeting peptide insertion at position 589; preferably wherein the targeting peptide is selected from the group consisting of: SEQ ID NO: 5, 21, 23, 25, 27, 29, 31, 33, 35 and 37; preferably SEQ ID NO: 5.

In some preferred embodiments, the modified AAV capsid protein comprises a sequence selected from the group consisting of the sequence SEQ ID NO: 6 and the variant sequences having at least 85%, 87%, 88%, 90%, 95%, 97%, 98% or 99% identity with said sequence, wherein said variant sequences comprise no mutations in the muscle-targeting peptide sequence and the 5 amino acid sequence; preferably the 10 amino acid sequence before and after the insertion site.

In some preferred embodiments, the modified AAV capsid protein has an increased tropism for muscle, in particular heart and/or skeletal muscle, and/or a decreased tropism for liver.

The invention also relates to a polynucleotide encoding the modified AAV capsid protein according to the present disclosure; preferably comprising the sequence SEQ ID NO: 7 or a sequence having at least 80%, 85%, 90%, 95%, 97%, 98% or 99% identity with said sequence.

The invention further relates to a recombinant plasmid comprising the polynucleotide according to the present disclosure.

Another aspect of the invention relates to an AAV vector particle packaging a gene of interest, which comprises the modified AAV capsid protein according to the present disclosure.

In some preferred embodiments, the gene of interest is selected from the group consisting of: therapeutic genes; genes encoding therapeutic proteins or peptides such as therapeutic antibodies or antibody fragments and genome editing enzymes; and genes encoding therapeutic RNAs such as interfering RNAs, guide RNAs for genome editing and antisense RNAs capable of exon skipping.

Another aspect of the invention relates to a pharmaceutical composition comprising a therapeutically effective amount of AAV vector particle according to the present disclosure, or cell stably transduced by said AAV vector particle according to the present disclosure.

The invention relates also to a pharmaceutical composition according to the present disclosure for use as a medicament in gene therapy, preferably for treating muscle diseases.

In some preferred embodiments, the pharmaceutical composition targets a gene responsible for a muscle disease chosen from Dystrophinopathies and Limb-girdle muscular dystrophies; preferably a gene selected from the group comprising: *DMD, CAPN3, DYSF, FKRP, DNAJB6, ANO5, SGCA, SGCB* and *SGCG.*

### DETAILED DESCRIPTION OF THE INVENTION

### Modified AAV capsid protein

The invention relates to a modified adeno-associated virus (AAV) capsid protein comprising a muscle-targeting peptide insertion between two consecutive amino acids of the capsid protein sequence and without any modification of the capsid protein amino acid sequence upstream and downstream of the insertion site, wherein the targeting peptide comprises the sequence SEQ ID NO: 1 (RGDLGLS) and a sequence of small amino-acids at the N-terminal and C-terminal ends of SEQ ID NO: 1, and wherein the insertion site is into the variable region IV, V or VIII of the AAV capsid protein sequence.

The sequence RGDLGLS corresponds to the sequence of the peptide named P1 in the prior art.

The modified AAV capsid protein according to the invention is a functional AAV capsid which is able to form recombinant AAV vector particles which transduce a cell, tissue or organ, in particular a cell tissue or organ of interest (target cell, tissue or organ) and express a transgene in said cell, tissue or organ, in particular target cell tissue or organ. Furthermore, the modified AAV capsid protein according to the invention has an improved tropism compared to the corresponding unmodified AAV capsid protein from which it is derived and to a corresponding modified AAV capsid protein according to the prior art (e.g., in which peptide P1 insertion modifies AAV capsid protein sequence upstream and downstream of the insertion site).

As used herein, the term "tropism" refers to the capacity of an AAV capsid protein present in a recombinant AAV vector particle, to transduce some particular type(s) of cell(s), tissue(s) or organ(s) (e.g, cellular or tissue tropism). The modified AAV capsid protein which has improved tropism may have an increased tropism for at least one target cell, tissue or organ and/or a decreased tropism (or detargeting) for at least one off-target cell, tissue or organ compared to an unmodified capsid or a modified capsid according to the prior art as disclosed above. An increased tropism refers in particular to a transgene expression level that is increased in at least one target cell, tissue or organ, by at least 1.5 fold, preferably 3, 5, 10, 50, 100 folds or more compared to unmodified AAV capsid protein and by at least 1.2; 1.3; 1.4; 1.5; 1.6; 1.7; 1.8; 1.9, 2 folds, preferably 1.3 folds or more compared to prior art modified AAV capsid protein. A detargeting refers in particular to a transgene expression level that is decreased in at least one off-target cell, tissue or organ, by at least 1.5 folds, preferably 3, 5, 10, 50, 100 folds or more compared to unmodified AAV capsid protein and by at least 1.5 folds, preferably 3, 5, 10 folds or more compared to prior art modified AAV capsid protein. As a result of its improved tropism, the modified AAV capsid protein according to the invention has an improved biodistribution. This means that it targets significantly better a defined tissue (target tissue), group of tissues (for example skeletal muscle and/or heart) or organ (target tissue(s) or organ) without increasing the targeting of other (non-target) tissues (e.g. improved specificity) and/or it targets a specific tissue (non-target or off-target tissue or organ) with a lower efficacy (tissue detargeting, for example liver detargeting), usually to reduce unwanted toxicities.

The tropism of the modified AAV capsid protein according to the invention for a particular type of cell, tissue or organ may be determined by measuring the ability of AAV vector particles comprising the modified AAV capsid protein to transduce said particular type of cell, tissue or organ or express a transgene in said particular type of cell, tissue or organ, using standard assays that are well-known in the art such as those disclosed in the examples of the present application. For example, vector transduction or transgene expression are determined by local or systemic administration of AAV vector particles carrying the modified AAV capsid protein in animal models such as mouse models that are well known in the art and disclosed in the examples of the present application. AAV vectors comprising the corresponding unmodified AAV capsid protein and the corresponding modified AAV capsid protein according to the prior are used for comparison. Vector transduction may be determined by measuring vector genome copy number per diploid genome by standard assays that are well known in the art such as real-time PCR assay. Transgene expression is advantageously measured using a reporter gene such as luciferase or fluorescent protein (GFP or others) by standard assays that are well known in the art such as *in vivo* or *in vitro* quantitative bioluminescence or fluorescence assays *in vivo* or *in vitro.*

In the following description, the amino acid residues are designated by the standard one letter amino acid code.

"a", "an", and "the" include plural referents, unless the context clearly indicates otherwise. As such, the term "a" (or "an"), "one or more" or "at least one" can be used interchangeably herein; unless specified otherwise, "or" means "and/or".

As used herein, the term "muscle" refers to cardiac muscle (i.e. heart) and skeletal muscle. The term "muscle cells" refers to myocytes, myotubes, myoblasts, and/or satellite cells. The skeletal muscles are classified in different groups based on their anatomical position in the body. The tropism of the hybrid AAV capsid according to the invention for different skeletal muscle groups may be measured in mice *Tibialis* (TA), *Extensor Digitorum Longus* (EDL), *Quadriceps* (Qua), *Gastrocnemius* (Ga), *Soleus* (Sol), Triceps, Biceps and/or Diaphragm; in particular in mice *Tibialis* (TA) and Diaphragm muscles. The expression "Tropism for muscle" refers to the tropism for the heart and the various skeletal muscles present in the body.

As used herein, a "muscle-targeting peptide" refers to a peptide which binds to muscle cells and directs or targets capsid-modified rAAV vectors carrying the peptide to muscle cells and tissue *in vivo.*

A modified AAV capsid protein according to the invention is a recombinant protein.

As used herein, "a sequence" refers to 1 amino acid or at least 2 consecutive amino acids.

In the present description, an insertion at a given position of AAV capsid protein sequence refers to an insertion after the amino acid residue at that position.

The term "identity" refers to the sequence similarity between two polypeptide molecules or between two nucleic acid molecules. When a position in both compared sequences is occupied by the same base or same amino acid residue, then the respective molecules are identical at that position. The percentage of identity between two sequences corresponds to the number of matching positions shared by the two sequences divided by the number of positions compared and multiplied by 100. Generally, a comparison is made when two sequences are aligned to give maximum identity. The identity may be calculated by alignment using, for example, the GCG (Genetics Computer Group, Program Manual for the GCG Package, Version 7, Madison, Wisconsin) pileup program, or any of sequence comparison algorithms such as BLAST, FASTA or CLUSTALW.

In some embodiments, the modified AAV capsid protein according to the invention has an increased tropism for muscle, in particular heart and/or skeletal muscle, and/or a decreased tropism for liver (liver detargeting). The tropism of the modified AAV capsid protein according to the invention is improved compared to the corresponding unmodified AAV capsid protein from which it is derived and to a corresponding modified AAV capsid protein in which muscle-targeting peptide insertion modifies AAV capsid protein sequence before and after the insertion site (modified AAV capsid of the prior art).

The muscle-targeting peptide consists generally of a sequence of up to 30 amino acids. The targeting peptide may consist of 9, 10, 11, 12, 13, 14, 15, 16, 17, 18, 19, 20, 21, 22, 23, 24, 25, 26, 27, 28, 29, 30 amino acids. In some embodiments, the muscle-targeting peptide consists of a sequence of up to 25, 20 or 15 amino acids. Preferably, the targeting peptide consists of a sequence of 12, 13, 14 or 15 amino acids.

The muscle-targeting peptide is inserted into a site exposed on the capsid surface which is the variable region IV, V or VIII of the AAV capsid protein sequence. The variable regions (VRs) VR-IV, -V and -VIII which form loops at the top of the protrusions are site exposed on the AAV capsid surface which are well-known in the art (Review in Büning et al., Molecular Therapy: Methods & Clinical Development, 2019, 12, 248-). VR-IV corresponds to Y445 to A476 (broad definition) or Q451 to L462 (narrow definition); VR-V corresponds to C485 to G515 (broad definition) or R490 to T509 (narrow definition); VR-VIII corresponds to I581 to L604 (broad definition) or L586 to I595 (narrow definition) according to the numbering in AAV8 capsid protein sequence. The peptide insertion site is advantageously at a site of the common VP3 region suitable exposed on the AAV capsid surface such as for example position 587, 588, 589, 453, 520, 584 and 585, according to the numbering in AAV2 capsid protein sequence. The peptide insertion sites are indicated by reference to AAV2 or AAV8 capsid amino acid sequence. After sequence alignment of any other AAV capsid sequence with AAV2 or AAV8 capsid sequence using standard protein sequence alignment programs that are well-known in the art, such as for example BLAST, FASTA, CLUSTALW, and the like, a person skilled in the art can easily obtained the corresponding positions of the peptide insertion sites in other AAV capsid sequences.

Preferred insertion sites for AAV serotypes include position 590 in AAV1; positions 587 or 588 in AAV2; position 586 in AAV3 or AAV4; position 575 in AAV5; position 585 in AAV6; positions 585 or 590 in AAV8; positions 588 or 589 in AAV9; position 589 in AAV9.rh74.

The modified AAV capsid protein may comprise one or more muscle-targeting peptide insertions at different sites of the AAV capsid protein.

According to the present invention, the sequence of small amino-acids in N-ter and C-ter end of SEQ ID NO: 1 is a flanking sequence directly adjacent to the N-ter and C-ter end of SEQ ID NO: 1. Small amino acids have a lateral chain with a small steric hindrance, generally chosen from H, or a C1-C2 alkyl group that may be substituted with one or more of methyl, carboxyl, hydroxyl and/or amine groups; preferably the lateral chain consists of C, H, N and O atoms and/or does not comprise cyclic groups. The small amino acid(s) may be neutral and/or flexible. Preferably, the small amino acids are selected from the group consisting of: alanine (A), glycine (G), serine(S), Asparagine (N), Threonine (T), Aspartic acid (D) and Leucine (L); more preferably A, G, S, T and L. Preferably, the flanking sequences consist of up to five amino acids (1, 2, 3, 4 or 5) which may be the same or different; more preferably 2 to five amino acids (2, 3, 4 or 5) which may be the same or different. The N-terminal and C-terminal flanking sequences are advantageously selected from the group consisting of: G, SG, TG AA, GG, SA, AG, GA, AAA, ,GST, SGS, SGA, AGA, GAA, and ALA; preferably G, GST, AAA, SGS, SGA, AGA, GAA or ALA in N-ter and G, SG, AA, TG, GG, SA, AG or GA in C-ter. In some more preferred embodiments, the muscle-targeting peptide comprises AAA at the N-terminal end and SG at the C-terminal end of SEQ ID NO: 1.

The modified AAV capsid protein may be derived from any natural or artificial AAV capsid serotype including hybrid serotypes and variant serotypes. Non-limiting examples of AAV capsid serotypes from which the modified AAV capsid protein may be derived include AAV1, AAV2, AAV3 (including types 3A and 3B), AAV4, AAV5, AAV6, AAV7, AAV8, AAV9, AAV10, AAV11, AAV12, AAV2i8, AAVrh10, AAVrh39, AAVrh43, AAVrh74, AAV-LK03, AAV2G9, AAV.PHP, AAV-Anc80, AAV3B and AAV9.rh74 (as disclosed in WO2019/193119). In some embodiments, the modified AAV capsid protein is from AAV serotype selected from the group consisting of: AAV6, AAV8, AAV9, AAVrh74 and AAV9.rh74. In some preferred embodiments, the modified AAV capsid protein is from AAV8, AAV9 or AAV9.rh74; or from AAV9 or AAV9.rh74, still more preferably AAV9.rh74. AAV9 capsid corresponds in particular to the amino acid sequence GenBank accession number AY530579.1 accessed on 24 June 2004. AAV9.rh74 capsid corresponds to the amino acid sequence SEQ ID NO: 2 which may be encoded by the CDS of SEQ ID NO: 3. The muscle-targeting peptide is advantageously inserted at position 585 or 590 of AAV8, position 588 or 589 of AAV9, or position 589 of AAV9.rh74.

In some preferred embodiments, the modified AAV capsid protein is from AAV9.rh74 and comprises the muscle-targeting peptide insertion at position 589 of AAV9.rh74 capsid protein.

In some preferred embodiments, the targeting peptide comprises or consists of a sequence selected from the group consisting of: AAARGDLGLSSG (SEQ ID NO: 5); AAARGDLGLSAA (SEQ ID NO: 21); GSTRGDLGLSTG (SEQ ID NO: 23); GRGDLGLSG (SEQ ID NO: 25); SGSRGDLGLSGG (SEQ ID NO: 27); SGARGDLGLSSA (SEQ ID NO: 29); SGARGDLGLSAG (SEQ ID NO: 31); AGARGDLGLSSG (SEQ ID NO: 33); GAARGDLGLSGA (SEQ ID NO: 35); and ALARGDLGLSAG (SEQ ID NO: 37); preferably SEQ ID NO: 5, 21, 23, 27, 29, 31, 33, 35 and 37; still more preferably SEQ ID NO: 5. In some more preferred embodiments, the targeting peptide comprising or consisting of any one of the preceding sequences is inserted in AAV8, AAV9 or AAV9.rh74 capsid; preferably AAV9.rh74 capsid. The targeting peptide peptide is advantageously inserted at position 590 of AAV8, position 588 of AAV9, or position 589 of AAV9.rh74.

In some more preferred embodiments, the modified AAV capsid protein, comprises or consists of a sequence selected from the group consisting of the sequence of SEQ ID NO: 6 and the sequences having at least 85%, 87%, 88%, 90%, 95%, 97%, 98% or 99% identity with said sequence; more preferably which comprises or consists of the sequence of SEQ ID NO: 6. The variant according to the invention has no mutations in the muscle-targeting peptide sequence and the 5 amino acid sequence; preferably the 10 amino acid sequence before and after the insertion site.

In some embodiments, the modified AAV capsid protein is a modified VP1, VP2 or VP3 protein. In some particular embodiments, the modified VP1, VP2 or VP3 protein is derived from SEQ ID NO: 6. VP2 corresponds to the amino acid sequence from T138 to the end of SEQ ID NO: 6. VP3 corresponds to the amino acid sequence from M204 to the end of SEQ ID NO: 6.

In some embodiments, the modified AAV capsid protein is a chimeric VP1 or VP2 protein comprising the muscle-targeting peptide insertion into the common VP3 region of an AAV serotype and VP1-specific and/or VP2-specific N-terminal region(s) from other AAV serotype(s) (e.g. AAV serotype(s) different from the serotype of the VP3 region). In some particular embodiments, the chimeric VP1 or VP2 protein is derived from SEQ ID NO: 6.

### Polynucleotide, vector, and use for AAV vector production

Another aspect of the invention is a polynucleotide encoding the recombinant modified AAV capsid protein in expressible form. The polynucleotide may be DNA, RNA or a synthetic or semi-synthetic nucleic acid.

In some embodiments, the polynucleotide encodes a modified AAV9.rh74 capsid protein according to the invention, in particular a modified AAV9.rh74 capsid protein comprising a targeting peptide comprising or consisting of a sequence selected from the group consisting of: AAARGDLGLSSG (SEQ ID NO: 5); AAARGDLGLSAA (SEQ ID NO: 21); GSTRGDLGLSTG (SEQ ID NO: 23); GRGDLGLSG (SEQ ID NO: 25); SGSRGDLGLSGG (SEQ ID NO: 27); SGARGDLGLSSA (SEQ ID NO: 29); SGARGDLGLSAG (SEQ ID NO: 31); AGARGDLGLSSG (SEQ ID NO: 33); GAARGDLGLSGA (SEQ ID NO: 35); and ALARGDLGLSAG (SEQ ID NO: 37); preferably SEQ ID NO: 5, 21, 23, 27, 29, 31, 33, 35 and 37; still more preferably SEQ ID NO: 5. The polynucleotide preferably comprises a sequence selected from the group consisting of SEQ ID NO: 7, 22, 24, 26, 28, 30, 32, 34, 36 and 38; more preferably SEQ ID NO: 7, 22, 24, 28, 30, 32, 34, 36 and 38; still more preferably SEQ ID NO: 7 .

In some preferred embodiments, the polynucleotide encodes a modified AAV9.rh74 capsid protein comprising or consisting of SEQ ID NO: 6 and the sequences having at least 85%, 87%, 88%, 90%, 95%, 97%, 98% or 99% identity with said sequence.

In some more preferred embodiments, the polynucleotide comprises the sequence SEQ ID NO: 7 or a sequence having at least 80%, 85%, 90%, 95%, 97%, 98% or 99% identity with said sequence. The sequence SEQ ID NO: 7 encodes the modified AAV9.rh74 capsid protein of SEQ ID NO: 6. The polynucleotide is a functional polynucleotide sequence, which means that the sequence of the polynucleotide codes for the modified AAV capsid protein according to the invention.

In some embodiments, the polynucleotide further encodes AAV Replicase (Rep) protein in expressible form, preferably Rep from AAV2.

The polynucleotide is advantageously inserted into a recombinant vector, which includes, in a non-limiting manner, linear or circular DNA or RNA molecules consisting of chromosomal, non-chromosomal, synthetic or semi-synthetic nucleic acids, such as in particular viral vectors, plasmid or RNA vectors. Numerous vectors into which a nucleic acid molecule of interest can be inserted in order to introduce it into and maintain it in a eukaryotic host cell are known *per se;* the choice of an appropriate vector depends on the use envisioned for this vector (for example, replication of the sequence of interest, expression of this sequence, maintaining of this sequence in extrachromosomal form, or else integration into the chromosomal material of the host), and also on the nature of the host cell.

In some embodiments, the vector is a plasmid.

The recombinant vector for use in the present invention is an expression vector comprising appropriate means for expression of the modified AAV capsid protein (AAV Cap), and maybe also AAV Rep protein. Usually, each coding sequence (modified AAV Cap and AAV Rep) is inserted in a separate expression cassette either in the same vector or separately. Each expression cassette comprises the coding sequence (open reading frame or ORF) functionally linked to the regulatory sequences which allow the expression of the corresponding protein in AAV producer cells, such as in particular promoter, promoter/enhancer, initiation codon (ATG), stop codon, transcription termination signal. Alternatively, the modified AAV Cap and the AAV Rep proteins may be expressed from a unique expression cassette using an Internal Ribosome Entry Site (IRES) inserted between the two coding sequences or a viral 2A peptide. In addition, the codon sequences encoding the modified AAV Cap, and AAV Rep if present, are advantageously optimized for expression in AAV producer cells, in particular human producer cells.

The vector, preferably a recombinant plasmid, or a cell stably transformed with the recombinant vector for expression of the modified AAV capsid protein, and preferably also of the AAV Rep protein are useful for producing hybrid AAV vectors comprising the hybrid AAV capsid protein of the invention, using standard AAV production methods that are well-known in the art (Review in Aponte-Ubillus et al., Applied Microbiology and Biotechnology, 2018, 102: 1045-1054). The cell stably expresses the modified AAV capsid and AAV Rep proteins (producer cell line). The producer cell is advantageously a human cell.

AAV vectors are usually produced by co-transfecting cells suitable for AAV production with a plasmid containing recombinant AAV vector genome comprising the gene of interest inserted in an expression cassette, flanked by AAV ITRs (AAV transfer plasmid), and plasmid(s) expressing AAV Rep and Cap proteins. Alternatively, producer cells according to the invention (which stably express AAV Rep and Cap proteins) may be transfected with an AAV transfer plasmid.

Briefly, following transfection with above plasmid(s) in the presence of sufficient helper function to permit packaging of the rAAV vector genome into AAV capsid particle, the cells are incubated for a time sufficient to allow the production of AAV vector particles, the cells are then harvested, lysed, and AAV vector particles are purified by standard purification methods such as affinity chromatography and Iodixanol or Cesium Chloride density gradient ultracentrifugation.

### AAV particle, cell

Another aspect of the invention is an AAV particle packaging a gene of interest comprising the modified recombinant AAV capsid protein of the invention.

The AAV particle comprises modified VP1, VP2 and/or VP3 capsid proteins according to the present invention. In some embodiments, the AAV particle comprises modified VP1, VP2 and VP3 capsid proteins of the same serotype. In some embodiments, the AAV particle further or alternatively comprises chimeric VP1 and/or VP2 capsid proteins and a modified VP3 protein according to the invention. In some embodiments, the AAV particle is a mosaic AAV particle further comprising another AAV capsid protein from a natural or artificial AAV serotype other than the serotype(s) of the modified AAV capsid including chimeric modified AAV capsid, wherein the mosaic AAV particle has an increased muscle tropism and/or a reduced liver tropism. An artificial AAV serotype may be with no limitation, a chimeric AAV capsid, a recombinant AAV capsid, or a humanized AAV capsid. Such an artificial capsid may be generated by any suitable technique, using a selected AAV sequence (e.g. a fragment of a VP1 capsid protein) in combination with heterologous sequences which may be obtained from a different selected AAV serotype, non-contiguous portions of the same AAV serotype, from a non-viral AAV source or from a non-viral source.

Preferably, the AAV particle is a recombinant AAV (rAAV) vector particle. The AAV vector particle is suitable for gene therapy directed to a target tissue or cells in the individual, in particular muscle cells or tissue. The rAAV vector particle is packaging a gene of interest. The genome of the rAAV vector may either be a single-stranded or self-complementary double-stranded genome (McCarty et al, Gene Therapy, 2003, Dec., 10(26), 2112-2118). Self-complementary vectors are generated by deleting the terminal resolution site (trs) from one of the AAV terminal repeats. These modified vectors, whose replicating genome is half the length of the wild-type AAV genome have the tendency to package DNA dimers. The AAV genome is flanked by ITRs. In particular embodiments, the AAV vector is a pseudotyped vector, *i.e.* its genome and capsid are derived from AAVs of different serotypes. In some preferred embodiments, the genome of the pseudotyped vector is derived from AAV2. The rAAV vector particle may be obtained using standard AAV production methods that are well-known in the art as disclosed above.

By "gene of interest", it is meant a gene useful for a particular application, such as with no limitation, diagnosis, reporting, modifying, therapy and genome editing.

For example, the gene of interest may be a therapeutic gene, a reporter gene or a genome-editing enzyme.

By "gene of interest for therapy", "gene of therapeutic interest", or "heterologous gene of interest", it is meant a therapeutic gene or a gene encoding a therapeutic protein, peptide or RNA.

The gene of interest is any nucleic acid sequence capable of modifying a target gene or target cellular pathway, in cells of target organs, in particular muscle. For example, the gene may modify the expression, sequence or regulation of the target gene or cellular pathway. In some embodiments, the gene of interest is a functional version of a gene or a fragment thereof. The functional version of said gene includes the wild-type gene, a variant gene such as variants belonging to the same family and others, or a truncated version, which preserves the functionality of the encoded protein at least partially. A functional version of a gene is useful for replacement or additive gene therapy to replace a gene, which is deficient or non-functional in a patient. In other embodiments, the gene of interest is a gene which inactivates a dominant allele causing an autosomal dominant genetic disease. A fragment of a gene is useful as recombination template for use in combination with a genome editing enzyme.

Alternatively, the gene of interest may encode a protein of interest for a particular application, (for example an antibody or antibody fragment, a genome-editing enzyme) or a RNA. In some embodiments, the protein is a therapeutic protein including a therapeutic antibody or antibody fragment, or a genome-editing enzyme. In some embodiments, the RNA is a therapeutic RNA.

In some embodiments, the sequence of the gene of interest is optimized for expression in the treated individual, preferably a human individual. Sequence optimization may include a number of changes in a nucleic acid sequence, including codon optimization, increase of GC content, decrease of the number of CpG islands, decrease of the number of alternative open reading frames (ARFs) and/or decrease of the number of splice donor and splice acceptor sites.

The gene of interest is a functional gene able to produce the encoded protein, peptide or RNA in the target cells of the disease, in particular muscle cells. In some embodiments, the gene of interest is a human gene. The AAV viral vector comprises the gene of interest in a form expressible in cells of target organs, in particular muscle cells, including cardiac and skeletal muscle cells muscles. In particular, the gene of interest is operably linked to appropriate regulatory sequences for expression of a transgene in the individual's target cells, tissue(s) or organ(s). Such sequences which are well-known in the art include in particular a promoter, and further regulatory sequences capable of further controlling the expression of a transgene, such as without limitation, enhancer, terminator, intron, silencer, in particular tissue-specific silencer, and microRNA. The gene of interest is operably linked to a ubiquitous, tissue-specific or inducible promoter which is functional in cells of target organs, in particular muscle(s). The gene of interest may be inserted in an expression cassette further comprising additional regulatory sequences as disclosed above.

Examples of ubiquitous promoters include the CAG promoter, phosphoglycerate kinase 1 (PGK) promoter, the cytomegalovirus enhancer/promoter (CMV), the SV40 early promoter, the retroviral Rous sarcoma virus (RSV) LTR promoter, the dihydrofolate reductase promoter, the β-actin promoter, and the EF1 promoter. Muscle-specific promoters include without limitation, upstream sequences derived from the desmin (Des) promoter, muscle creatine kinase (MCK) promoter, , alpha-myosin heavy chain (alpha-MHC) promoter, myosin light chain 2 (MLC-2) promoter, cardiac troponin C (cTnC) promoter, the human skeletal actin (HSA) promoter or synthetic muscle-specific promoters such as SpC5-12 promoter, CK6 promoter.

The RNA is advantageously complementary to a target DNA or RNA sequence or binds to a target protein. For example, the RNA is an interfering RNA such as a shRNA, a microRNA, a guide RNA (gRNA) for use in combination with a Cas enzyme or similar enzyme for genome editing, an antisense RNA capable of exon skipping such as a modified small nuclear RNA (snRNA) or a long non-coding RNA. The interfering RNA or microRNA may be used to regulate the expression of a target gene involved in muscle disease. The guide RNA in complex with a Cas enzyme or similar enzyme for genome editing may be used to modify the sequence of a target gene, in particular to correct the sequence of a mutated/deficient gene or to modify the expression of a target gene involved in a disease, in particular a neuromuscular disease. The antisense RNA capable of exon skipping is used in particular to correct a reading frame and restore expression of a deficient gene having a disrupted reading frame. In some embodiments, the RNA is a therapeutic RNA.

The genome-editing enzyme according to the invention is any enzyme or enzyme complex capable of modifying a target gene or target cellular pathway, in particular in muscle cells. For example, the genome-editing enzyme may modify the expression, sequence or regulation of the target gene or cellular pathway.The genome-editing enzyme is advantageously an engineered nuclease, such as with no limitations, a meganuclease, zinc finger nuclease (ZFN), transcription activator-like effector-based nuclease (TALENs), Cas enzyme from clustered regularly interspaced palindromic repeats (CRISPR)-Cas system and similar enzymes. The genome-editing enzyme, in particular an engineered nuclease such as Cas enzyme and similar enzymes, may be a functional nuclease which generates a double-strand break (DSB) or single-stranded DNA break (nickase such as Cas9(D10A) in the target genomic locus and is used for site-specific genome editing applications, including with no limitations: gene correction, gene replacement, gene knock-in, gene knock-out, mutagenesis, chromosome translocation, chromosome deletion, and the like. For site-specific genome editing applications, the genome-editing enzyme, in particular an engineered nuclease such as Cas enzyme and similar enzymes may be used in combination with a homologous recombination (HR) matrix or template (also named DNA donor template) which modifies the target genomic locus by double-strand break (DSB)-induced homologous recombination. In particular, the HR template may introduce a transgene of interest into the target genomic locus or repair a mutation in the target genomic locus, preferably in an abnormal or deficient gene causing a a muscle disorder, such as for example a neuromuscular disease. Alternatively, the genome-editing enzyme, such as Cas enzyme and similar enzymes may be engineered to become nuclease-deficient and used as DNA-binding protein for various genome engineering applications such as with no limitation: transcriptional activation, transcriptional repression, epigenome modification, genome imaging, DNA or RNA pull-down and the like.

The invention also relates to an isolated cell, in particular a cell from an individual, which is stably transduced with a rAAV vector particle of the invention. The individual is advantageously a patient to be treated. In some embodiments, the cell is a muscle cell according to the present disclosure, a progenitor of said cell or a pluripotent stem cell such as induced pluripotent stem cell (iPS cell), embryonic stem cells, fetal stem cell and adult stem cell.

### Pharmaceutical composition and therapeutic uses

Another aspect of the invention is a pharmaceutical composition comprising at least an active agent selected from an AAV vector particle of the invention, or cell stably transduced by said AAV vector particle, and a pharmaceutically acceptable carrier.

The pharmaceutical composition of the invention may be used for treating diseases by gene therapy, in particular targeted gene therapy directed to muscle cells or tissue. The pharmaceutical composition of the invention comprising the cell may be used for treating diseases by cell therapy, in particular cell therapy directed to muscle cells.

As used herein "Gene therapy" refers to a treatment of an individual which involves delivery of nucleic acid of interest into an individual's cells for the purpose of treating a disease. Delivery of the nucleic acid is generally achieved using a delivery vehicle, also known as a vector. The rAAV vector particle of the invention may be employed to deliver a gene to a patient's cells.

As used herein "Cell therapy" refers to a process wherein cells stably transduced by a rAAV vector particle of the invention are delivered to the individual in need thereof by any appropriate mean such as for example by intravenous injection (infusion), or injection in the tissue of interest (implantation or transplantation). In particular embodiments, cell therapy comprises collecting cells from the individual, transducing the individual's cells with the rAAV vector particle of the invention, and administering the stably transduced cells back to the patient. As used herein "cell" refers to isolated cell, natural or artificial cellular aggregate, bioartificial cellular scaffold and bioartificial organ or tissue.

Gene therapy can be performed by gene transfer, gene editing, exon skipping, RNA-interference, trans-splicing or any other genetic modification of any coding or regulatory sequences in the cell, including those included in the nucleus, mitochondria or as commensal nucleic acid such as with no limitation viral sequences contained in cells.

The two main types of gene therapy are the following:
- a therapy aiming to provide a functional replacement gene for a deficient/abnormal gene: this is replacement or additive gene therapy;
- a therapy aiming at gene or genome editing: in such a case, the purpose is to provide to a cell the necessary tools to correct the sequence or modify the expression or regulation of a deficient/abnormal gene so that a functional gene is expressed or an abnormal gene is suppressed (inactivated): this is gene editing therapy.

In additive gene therapy, the gene of interest may be a functional version of a gene, which is deficient or mutated in a patient, as is the case for example in a genetic disease. In such a case, the gene of interest will restore the expression of a functional gene. Thus, by gene editing or gene replacement a correct version of this gene is provided in target cells, in particular muscle cells of affected patients, this may contribute to effective therapies against the disease.

Gene or genome editing uses one or more gene(s) of interest, such as:
- a gene encoding a therapeutic RNA as defined above such as an interfering RNA like a shRNA or a microRNA, a guide RNA (gRNA) for use in combination with a Cas enzyme or similar enzyme, or an antisense RNA capable of exon skipping such as a modified small nuclear RNA (snRNA); and
- a gene encoding a genome-editing enzyme as defined above such as an engineered nuclease like a meganuclease, zinc finger nuclease (ZFN), transcription activator-like effector-based nuclease (TALENs), Cas enzyme or similar enzymes; or a combination of such genes, and maybe also a fragment of a functional version of a gene for use as recombination template, as defined above.

Gene therapy is used for treating muscle diseases. Muscle diseases include various inherited (genetic) and acquired diseases or disorders affecting the structure or function of muscle including skeletal and cardiac muscle. The diseases may be caused by trauma, infection, degeneration, structural or metabolic defects, tumors, inflammatory or autoimmune disorders, or other causes. Non-limiting examples of muscle diseases that can be treated by gene therapy include neuromuscular genetic disorders such as muscular genetic disorders; cancer and auto-immune diseases.

In some embodiments, the target gene for gene therapy (additive gene therapy or gene editing) is a gene responsible for a neuromuscular disease. Neuromuscular genetic disorders include in particular: Muscular dystrophies, Congenital muscular dystrophies, Congenital myopathies, Distal myopathies, Other myopathies, Myotonic syndromes, Ion Channel muscle diseases, Malignant hyperthermia, Metabolic myopathies, Hereditary Cardiomyopathies, Congenital myasthenic syndromes, Motor Neuron diseases, Hereditary paraplegia, Hereditary motor and sensory neuropathies and other neuromuscular disorders.

Particular examples of neuromuscular genetic disorders that can be treated using a capsid-modified rAAV according to the invention are listed below:
- Dystrophinopathies are a spectrum of X-linked muscle diseases caused by pathogenic variants in *DMD* gene, which encodes the protein dystrophin. Dystrophinopathies comprises Duchenne muscular dystrophy (DMD), Becker muscular dystrophy (BMD) and DMD-associated dilated cardiomyopathy.
- The Limb-girdle muscular dystrophies (LGMDs) are a group of disorders that are clinically similar to DMD but occur in both sexes as a result of autosomal recessive and autosomal dominant inheritance. Limb-girdle dystrophies are caused by mutation of genes that encode sarcoglycans and other proteins associated with the muscle cell membrane, which interact with dystrophin. The term LGMD1 refers to genetic types showing dominant inheritance (autosomal dominant), whereas LGMD2 refers to types with autosomal recessive inheritance. Pathogenic variants at more than 50 loci have been reported (LGMD1A to LGMD1G; LGMD2A to LGMD2W).Calpainopathy (LGMD2A) is caused by mutation of the gene *CAPN3* with more than 450 pathogenic variants described. Contributing genes to LGMD phenotype include: anoctamin 5 (*ANO5*), blood vessel epicardial substance *(EVES),* calpain 3 *(CAPN3),* caveolin 3 (*CAV3*), CDP-L-ribitol pyrophosphorylase A *(CRPPA),* dystroglycan 1 (*DAG1*), desmin *(DES),* DnaJ heat shock protein family (Hsp40) homolog, subfamily B, member 6 (*DNAJB6*), dysferlin *(DYSF),* fukutin related protein *(FKRP),* fukutin *(FKT),* GDP-mannose pyrophosphorylase B *(GMPPB),* heterogeneous nuclear ribonucleoprotein D like *(HNRNPDL),* LIM zinc finger domain containing 2 *(LIMS2),* lain A:C *(LMNA),* myotilin *(MYOT),* plectin *(PLEC),* protein O-glucosyltransferase 1 (*PLOGLUT1*), protein O-linked mannose N-acetylglucosaminyltransferase 1 (beta 1,2-) (*POMGNT1*), protein O-mannose kinase *(POMK),* protein O-mannosyltransferase 1 (*POMT1*), protein O-mannosyltransferase *2* (*POMT2*), sarcoglycan alpha *(SGCA),* sarcoglycan beta *(SGCB),* sarcoglycan delta *(SGCD),* sarcoglycan gamma *(SGCG),* titin-cap *(TCAP),* transportin 3 (*TNPO3*), torsin 1A interacting protein (*TOR1AIP1*), trafficking protein particle complex 11 (*TRAPPC11*), tripartite motif containing 32 (TRIM 32) and titin (TTN). Major contributing genes to LGMD phenotype include *CAPN3, DYSF, FKRP* and *ANO5* (Babi Ramesh Reddy Nallamilli et al., Annals of Clinical and Translational Neurology, 2018, 5, 1574-1587.
- The Emery-Dreifuss Muscular Dystrophy (EDMD) caused by defects in one of the gene including the *EMD* gene (coding for emerin), the *FHL1* gene and the *LMNA* gene (encoding lamin A and C).
- Nesprin-1 and Nesprin-2 related muscular dystrophy caused by defects in the *SYNE1* and *SYNE2* gene, respectively; LUMA related muscular dystrophy caused by defects in the *TMEM43* gene; LAP1B related muscular dystrophy caused by defects in the *TOR1AIP1* gene.
- Facio-scapulo-humeral muscular dystrophy, type 1 (FSHD1A), such as associated with defect in the *DUX4* gene (contraction of the D4Z4 macrosatellite repeat in the subtelomeric region of chromosome 4q35) or the *FRG1* gene; Facio-scapulo-humeral muscular dystrophy, type 2 (FSHD1B) caused by defects in the *SMCHD1* gene.
- Spinal muscular atrophy is a genetic disorder caused by mutations in the Survival Motor Neuron 1 (*SMN1*) gene which is characterized by weakness and wasting (atrophy) in muscles used for movement.
- X-linked myotubular myopathy is a genetic disorder caused by mutations in the myotubularin (*MTM1*) gene which affects muscles used for movement (skeletal muscles) and occurs almost exclusively in males. This condition is characterized by muscle weakness (myopathy) and decreased muscle tone (hypotonia).
- Titinopathies are genetic disorders caused by mutations in the Titin (TTN) gene. Both dominant and recessive *TTN* mutations have been reported to cause a wide spectrum of cardiac and skeletal muscle diseases. Dominant titinopathies include hereditary myopathy with early respiratory failure (HMERF) caused by mutations in exon 344, and late-onset tibial muscular dystrophy (TMD). Recessive titinopathies include limb-girdle muscular dystrophy 2J, young- or early-adult-onset distal titinopathy, Emery-Dreifuss-like myopathy without cardiomyopathy, and congenital myopathy with or without heart disease.
- Pompe disease is a genetic disorder caused by mutations in the acid alpha-glucosidase *(GAA)* gene. Mutations in the *GAA* gene prevent acid alpha-glucosidase from breaking down glycogen effectively, which allows this sugar to build up to toxic levels in lysosomes. This buildup damages organs and tissues throughout the body, particularly the muscles, leading to the progressive signs and symptoms of Pompe disease.
- Glycogen storage disease III (GSD3) is an autosomal recessive metabolic disorder caused by homozygous or compound heterozygous mutation in the Amylo-Alpha-1, 6-Glucosidase, 4-Alpha-Glucanotransferase (AGL) gene which encodes the glycogen debrancher enzyme and associated with an accumulation of abnormal glycogen with short outer chains. Clinically, patients with GSD III present in infancy or early childhood with hepatomegaly, hypoglycemia, and growth retardation. Muscle weakness in those with IIIa is minimal in childhood but can become more severe in adults; some patients develop cardiomyopathy.

In some embodiments, the target gene for gene therapy (additive gene therapy or gene editing) is a gene responsible for a neuromuscular disease selected from the group comprising Dystrophinopathies *(DMD* gene) and Limb-girdle muscular dystrophies (LGMDs) *(CAPN3, DYSF, FKRP, ANO5, DNAJB6* genes and others such as *SGCA, SGCB, SGCG).* In some preferred embodiments, the target gene for gene therapy is selected from the group consisting of: *DMD, CAPN3, DYSF, FKRP, DNAJB6, ANO5, SGCA, SGCB* and *SGCG.*

A specific example of gene editing would be the treatment of Limb-girdle muscular dystrophy 2A (LGMD2A) which is caused by mutations in the calpain-3 gene *(CAPN3).* Other non-limiting examples would be the treatment of mutations in the *DMD* or *TNT* genes.

Thus, by gene editing or gene replacement a correct version of this gene is provided in muscle cells of affected patients, this may contribute to effective therapies against this disease. Other genetic diseases of the muscle as listed above could be treated by gene replacement or gene editing using the same principle.

Replacement or additive gene therapy may be used to treat cancer, in particular rhabdomyosarcomas. Genes of interest in cancer could regulate the cell cycle or the metabolism and migration of the tumor cells, or induce tumor cell death. For instance, inducible caspase-9 could be expressed in muscle cells to trigger cell death, preferably in combination therapy to elicit durable anti-tumor immune responses.

Gene editing may be used to modify gene expression in target cells, in particular muscle cells, in the case of auto-immunity or cancer, or to perturb the cycle of viruses in such cells. In such cases, preferably, the gene of interest is chosen from those encoding guide RNA (gRNA), site-specific endonucleases (TALEN, meganucleases, zinc finger nucleases, Cas nuclease), DNA templates and RNAi components, such as shRNA and microRNA. Tools such as CRISPR/Cas9 may be used for this purpose.

In some embodiments, gene therapy is used for treating diseases affecting other tissues, by expression of a therapeutic gene in target tissue, in particular, muscle tissue. This is useful to avoid expression of the therapeutic gene in the liver, in particular in patients having a concurrent hepatic disorder such as hepatitis. The therapeutic gene encodes preferably a therapeutic protein, peptide or antibody which is secreted from the muscle cells into the blood stream where it can be delivered to other target tissues such as for example the liver. Examples of therapeutic genes include with no limitation: Factor VIII, Factor IX and GAA genes.

The pharmaceutical composition of the invention which comprises AAV vector particles with reduced liver tropism may be administered to patients having concurrent liver disease such as for example hepatitis including viral or toxic hepatitis.

In the various embodiments of the present invention, the pharmaceutical composition comprises a therapeutically effective amount of rAAV vector particle or cell. In the context of the invention a therapeutically effective amount refers to a dose sufficient for reversing, alleviating or inhibiting the progress of the disorder or condition to which such term applies, or reversing, alleviating or inhibiting the progress of one or more symptoms of the disorder or condition to which such term applies. The term "effective dose" or "effective dosage" is defined as an amount sufficient to achieve, or at least partially achieve, the desired effect.

The effective dose is determined and adjusted depending on factors such as the composition used, the route of administration, the physical characteristics of the individual under consideration such as sex, age and weight, concurrent medication, and other factors, that those skilled in the medical arts will recognize.

In the various embodiments of the present invention, the pharmaceutical composition comprises a pharmaceutically acceptable carrier and/or vehicle.

A "pharmaceutically acceptable carrier" refers to a vehicle that does not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

Preferably, the pharmaceutical composition contains vehicles, which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of inj ectable solutions.

The pharmaceutical forms suitable for injectable use include sterile aqueous solutions or suspensions. The solution or suspension may comprise additives which are compatible with viral vectors and do not prevent viral vector particle entry into target cells. In all cases, the form must be sterile and must be fluid to the extent that easy syringe ability exists. It must be stable under the conditions of manufacture and storage and must be preserved against the contaminating action of microorganisms, such as bacteria and fungi. An example of an appropriate solution is a buffer, such as phosphate buffered saline (PBS) or Ringer lactate.

A further aspect of the invention relates to a pharmaceutical composition according to the present disclosure as medicament and/or for use in gene therapy, in particular for use in the treatment of a muscle disorder, in particular neuromuscular genetic disease according to the present disclosure.

As used herein, the term "patient" or "individual" includes human and other mammalian subjects that receive either prophylactic or therapeutic treatment. Preferably, a patient or individual according to the invention is a human.

Treatment", or "treating" as used herein, is defined as the application or administration of a therapeutic agent or combination of therapeutic agents to a patient, or application or administration of said therapeutic agents to an isolated tissue or cell line from a patient, who has a disease, in particular a muscle disorder with the purpose to cure, heal, alleviate, relieve, alter, remedy, ameliorate, improve or affect the disease, or any symptom of the disease. In particular, the terms "treat' or treatment" refers to reducing or alleviating at least one adverse clinical symptom associated with the disease.

The term "treatment" or "treating" is also used herein in the context of administering the therapeutic agents prophylactically.

The pharmaceutical composition of the present invention is generally administered according to known procedures, at dosages and for periods of time effective to induce a therapeutic effect in the patient. The pharmaceutical composition may be administered by any convenient route, such as in a non-limiting manner by infusion or bolus injection, by absorption through epithelial or mucocutaneous linings (e.g., oral mucosa, rectal and intestinal mucosa, etc.). The administration can be systemic, local or systemic combined with local; systemic includes parenteral and oral, and local includes local and loco-regional. Systemic administration is preferably parenteral such as subcutaneous (SC), intramuscular (IM), intravascular such as intravenous (IV) or intraarterial; intraperitoneal (IP); intradermal (ID), epidural or else. The administration may be for example by injection or perfusion. In some preferred embodiments, the administration is parenteral, preferably intravascular such as intravenous (IV) or intraarterial. The parenteral administration is advantageously by injection or perfusion.

The various embodiments of the present disclosure can be combined with each other and the present disclosure encompasses the various combinations of embodiments of the present disclosure.

The practice of the present invention will employ, unless otherwise indicated, conventional techniques, which are within the skill of the art. Such techniques are explained fully in the literature.

The invention will now be exemplified with the following examples, which are not limitative, with reference to the attached drawings in which:

### FIGURE LEGENDS

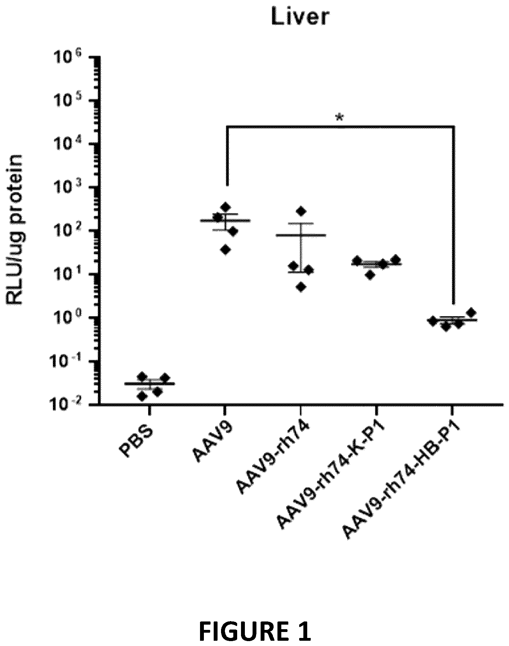

**Figure 1****: Comparison of luciferase transgene expression in liver of mice injected with AAV9.rh74 modified capsids**
   rAAV vectors with modified AAV9.rh74 capsid: AAV9-rh74-HB-P1 (HB-P1) according to the invention and AAV9-rh74-K-P1 (K-P1) according to the prior art and controls (AAV9; AAV9-rh74) were injected intravenously (4×10¹⁰ vg/mouse) in 5 weeks old B6 albino male mice (n=4). Luciferase expression was analyzed in liver by in vitro luciferase assay of liver tissue lysate. Data are shown as the mean ± SEM of the Relative Luminescence Units (RLU) per µg of protein (n=4). Statistical analysis was performed by Dunnett's multiple comparisons test (* p-value ≤ 0.05).

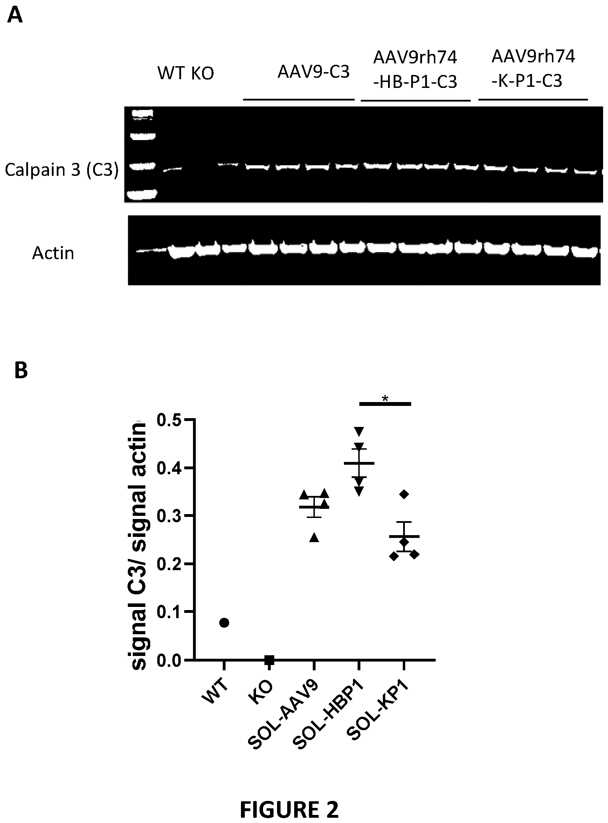

**Figure 2****: Comparison of Calpain 3 transgene expression in muscle of rat injected with AAV9.rh74 modified capsids**
   rAAV vectors expressing Calpain 3 (C3) transgene under the control of a muscle specific promoter and harboring a modified AAV9.rh74 capsid according to the invention (AAV9-rh74-HB-P1-C3) or according to the prior art (AAV9-rh74-K-P1-C3) and AAV9 control (AAV9-C3) were injected intravenously (4×10¹⁰ vg/rat) in 1 month-old rat. Calpain 3 expression was analyzed by *in vitro* Western blot assay of muscle biopsies using actin as internal control. A. Western Blot of Soleus muscle (SOL). **B.** Relative signal of Calpain 3 compared to actin in soleus muscle. WT: Normal rat. KO: Calpain 3 deficient rat.

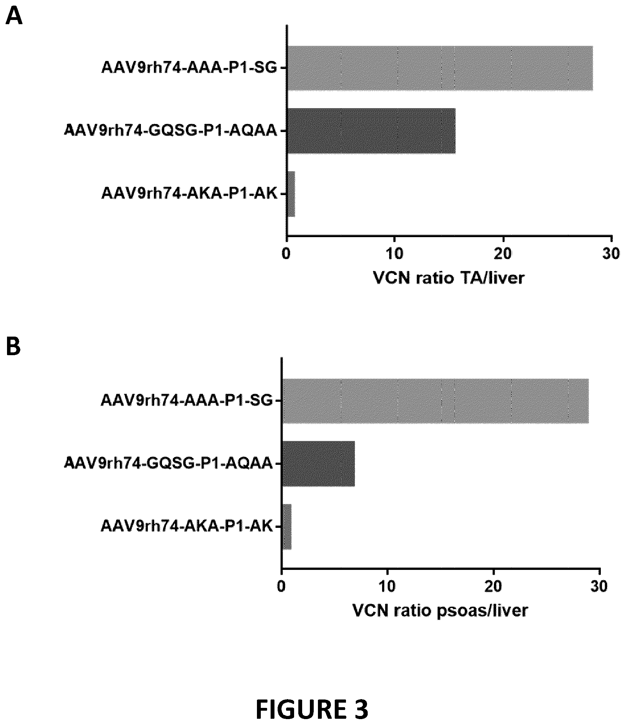

**Figure 3****: Comparison of vector genome copy number ratio in muscle to liver of mice injected with AAV9.rh74 modified capsids**
   rAAV vectors with modified AAV9.rh74 capsid: AAV9-rh74-AAA-P1-SG (HB-P1) according to the invention; AAV9-rh74-GQSG-P1-AQAA (K-P1) according to the prior art; and negative control AAV9-rh74-AKA-P1-AK were injected intravenously (1×10¹¹ vg/mouse) in 5 weeks old B6 albino male mice (n=4). Vector genome copy number (VCN) was quantified in muscle (Tibialis anterior (TA); psoas) and liver by multiplex qPCR and VCN ratio in muscle to liver was determined. **A.** VCN ratio TA/liver. **B.** VCN ratio psoas/liver.

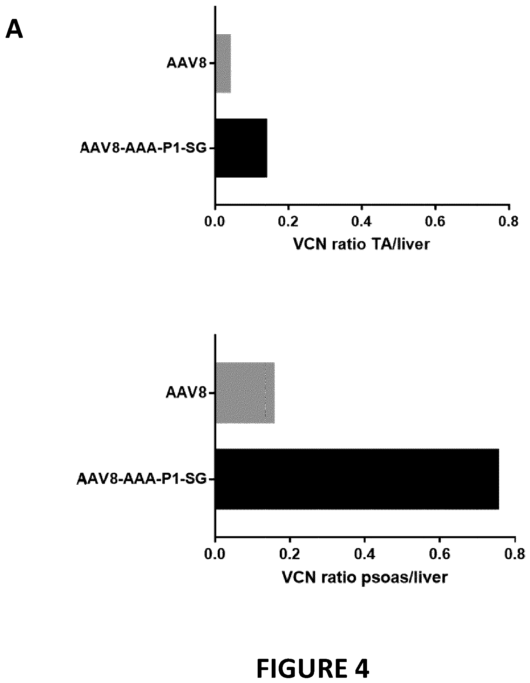

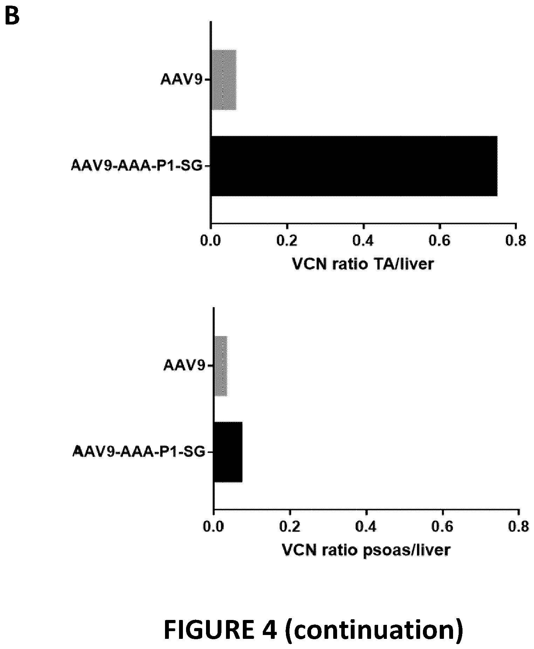

**Figure 4****: Comparison of vector genome copy number ratio in muscle to liver of mice injected with AAV9 and AAV8 modified capsids**
   rAAV vectors with AAV8 and AAV9 modified capsid according to the invention (AAV8-AAA-P1-SG and AAV9-AAA-P1-SG) and controls (AAV9, AAV8) were injected intravenously (1×10¹¹ vg/mouse) in 5 weeks old B6 albino male mice (n=4). Vector genome copy number (VCN) was quantified in muscle (Tibialis anterior (TA); psoas) and liver by multiplex qPCR and VCN ratio in muscle to liver was determined. **A.** P1 modified AAV8 capsid according to the invention. **B.** P1 modified AAV9 capsid according to the invention.

### EXAMPLE : Construction and biodistribution of the capsid-modified rAAV vector

Peptide P1 modified AAV capsids were constructed according to the invention, without modification of the AAV capsid amino acid sequence upstream and downstream of the insertion site (herein named as HB-P1). For comparison, peptide P1 modified AAV capsids were constructed according to the prior art, with modification of the AAV capsid amino acid sequence upstream and/or downstream of the insertion site (herein named as K-P 1).

### 1. Material and Methods

### 1.1. Plasmid construction

Plasmids containing hybrid AAV9.rh74 capsid modified by peptide P1 were derived from plasmid pRep2-Cap9rh74 containing AAV2 Rep and hybrid AAV9.rh74 capsid (Cap9rh74) genes disclosed in WO 2019/193119. Plasmid pRep2-Cap9rh74-HB-P1 encodes a modified hybrid AAV9.rh74 capsid (Cap9rh74-HB-P1 or Cap9rh74-AAA-P1-SG) comprising P1 peptide sequence flanked by AAA and SG (AAARGDLGLSSG; SEQ ID NO: 5) inserted at position 589 (e.g. between N589 and A590) of hybrid AAV9.rh74 capsid. Control Plasmid pRep2-Cap9rh74-AKA-P1-AK encodes a modified hybrid AAV9.rh74 capsid (Cap9rh74-AKA-P1-AK) comprising P1 peptide sequence flanked by non-small amino acid sequences AKA and AK, respectively in N-ter and C-ter (AKARGDLGLSAK; SEQ ID NO: 39) inserted at position 589 (e.g. between N589 and A590) of hybrid AAV9.rh74 capsid.

Plasmid pRep2-Cap9-HB-P1 (Cap9-AAA-P1-SG) encodes a modified hybrid AAV9 capsid (Cap9-HB-P1 or Cap9-AAA-P1-SG) comprising P1 peptide sequence flanked by AAA and SG (AAARGDLGLSSG; SEQ ID NO: 5) inserted at position 588 (e.g. between Q588 and A589) of AAV9 capsid. Plasmid pRep2-Cap8-HB-P1 (Cap8-AAA-P1-SG) encodes a modified hybrid AAV8 capsid (Cap8-HB-P1 or Cap8-AAA-P1-SG) comprising P1 peptide sequence flanked by AAA and SG (AAARGDLGLSSG; SEQ ID NO: 5) inserted at position 590 (e.g. between N590 and T591) of AAV8 capsid.

Plasmid pRep2-Cap9rh74-K-P1 encoding hybrid AAV9.rh74 capsid modified by peptide P1 (Cap9rh74-K-P1 or Cap9rh74-GQSG-P1-AQAA) obtained according to the peptide insertion strategy into AAV capsid disclosed in Kienle EC (Dissertation, 2014; precited) is used as control for comparison with pRep2-Cap9rh74-HB-P1 according to the invention. Cap9rh74-K-P1 construct comprises P1 peptide sequence inserted at position 589 of hybrid AAV9.rh74 capsid, according to the strategy described in WO19207132. Said strategy consists to firstly modify flanking sequences GQSG and AQAA, replacing the initial ⁵⁸⁷QQN⁵⁸⁹ and ⁵⁹⁰AAP⁵⁹² sequences flanking the insertion site in the hybrid AAV9.rh74 capsid and secondly insert P1 peptide. Therefore, Cap9rh74-K-P1 construct comprises the P1 containing sequence GQSGRGDLGLSAQAA (SEQ ID NO: 9) inserted between Q586 and I593 of hybrid AAV9.rh74 capsid.

*MscI* fragments containing the P1 peptide insertion were synthesized for each Cap9rh74 construct (GENEWIZ); Cap9rh74-HB-P1 (SEQ ID NO: 10), Cap9rh74-K-P1 (SEQ ID NO: 11) and Cap9rh74-AKA-P1-AK (SEQ ID NO: 40) and cloned into pUC plasmids. *AfeI-BsiWI* fragments containing the P1 peptide insertion were synthesized for the Cap9 and Cap8 constructs (GENEWIZ); Cap9-HB-P1 (SEQ ID NO: 41) and Cap8-HB-P1 (SEQ ID NO: 42). The fragments were then subcloned using InFusion cloning system, to replace the corresponding region in pRep2-Cap9rh74, pRep2-Cap9, and pRep2-Cap8 plasmids. Bacterial clones were sequence validated and stored as glycerols at -80°C and as purified plasmids at - 20°C. Cap9rh74-HB-P1 gene has the nucleotide sequence SEQ ID NO: 7 and codes for the AAV9-rh74-HB-P1 capsid protein of amino acid sequence SEQ ID NO: 6. Cap9rh74-K-P1 gene has the nucleotide sequence SEQ ID NO: 12 and codes for the AAV9-rh74-K-P1 capsid protein of amino acid sequence SEQ ID NO: 14.

### 1.2. rAA Vproduction

Recombinant AAVs (rAAVs) comprising the modified and non-modified AAV9rh74, AAV9, AAV8 capsids, and AAV9, AAV9rh74, AAV8 controls, containing GFP-Luciferase transgene under control of CMV promoter or calpain 3 under the control of a muscle specific promoter were produced in Généthon as described in Ayuso E. et al. (Hum. Gene Ther. 2014, 25, 977-987). Viral genomes are quantified as described in Rohr et al. (J. Virol. Methods, 2002, 106, 81-88). rAAV titers are expressed as viral genome copy number (vg).

### 1.3 In vivo experiment

AAV9rh74Animals were handled according to French and European legislation. The procedures on animals were approved by the local ethics committee and by the Ministry of Higher Education, Research and Innovation (APAFIS#19736). The rAAVs expressing GFP-luciferase were administered to 5 week-old B6 albino mice (n=4 or 5 per group) at a dose of 4×10¹² vg/kg or 1×10¹³ vg/kg, by intravenous (IV) injection. Acquisition of *in vivo* bioluminescence images was done 14 days post-injection. 3 weeks after injection, mice were sacrificed by cervical dislocation and several muscles and organs were sampled : Tibialis anterior, Psoas, Diaphragm, heart, liver, kidneys, lung and adrenal gland. The rAAVs expressing calpain 3 (AAV9-ms-C3 (9-C3), AAV9-rh74-K-P1_C3 (K-P1-C3); AAV9-rh74-HBP1_C3 (HBP1-C3) were administered to 1 month-old Sprague Dawley rat (n=4 per group) at the dose of 1xe14vg/kg by intravenous injection. Two months after injection, soleus muscles were sampled.

### 1.4 In vivo bioluminescence

Acquisition of *in vivo* bioluminescence images was done 14 days post-injection. Mice were anesthetized by inhalation of isoflurane and injected intraperitoneally with 100 ul of 50 mg/ml D-luciferin (LifeTechnologies, California, USA). *In vivo* imaging was performed using an IVIS^{®} Lumina Imaging system (PerkinElmer).

### 1.5 Vector copy number quantification

gDNA and viral DNA were extracted from the different muscles and organs sampled using the NucleoMag Pathogen kit (Macherey-Nagel) and the KingFisher Flex instrument (ThermoFisher Scientific).

Viral genome copies were quantified by multiplex qPCR in a Light Cycler 480 Instrument (Roche), using Thermo Scientific Absolute qPCR ROX Mix, primers (Forward: CATCAATGGGCGTGGATAGC (SEQ ID NO: 15); Reverse: GGAGTTGTTACGACATTTTGGAAA (SEQ ID NO: 16)) and probe (ATTTCCAAGTCTCCACCC, FAM (SEQ ID NO: 17)) to detect CMV promoter from the vectors and primers (Forward: CTCCAAGCAGATGCAGCAGA (SEQ ID NO: 18); Reverse: ATAGCCTTGCGCATCATGGT (SEQ ID NO: 19)) and probe (CCGTGGTGCTGATGGGCAAGAA, VIC (SEQ ID NO: 20)) to detect Rplp0 (60S acidic ribosomal protein P0) as internal control of the sample.

### 1.6. In vitro luciferase assay

Samples were homogenized in a lysis buffer (25 mM Tris-phosphate, 15% glycerol, 1mM DTT, 1mM EDTA, 8mM MgCl2, 0.2% Triton X-100 ) with protease inhibitor cocktail (cOmpleteTM ULTRA Tablets, Mini EDTA-free, EASYpack Protease Inhibitor Cocktail Tablets REF:5892791001) using a FastPrep-24 Classic Instrument (MP Biomedicals) (5m/s, 40s). Samples were subjected to 3 freezing/thawing cycles. After centrifugation (5 min, 10000 g, +4°C), 10 ul of supernatant were transferred to a white opaque plate. An EnSpire Multimode Plate Reader (PerkinElmer), with a pumping system that allows dispatching of 100 ul of the assay buffer (same as lysis buffer without Triton X-100 and with 2nM ATP, Sigma, REF: 10519979001) and 100 ul of 167 uM D-Luciferin, was used to measure the luminescence signal from the samples homogenates. Quantification of proteins, to normalize the luminescence signal by the quantity of proteins of the sample, was performed using the Pierce BCA Protein Assay kit.

### 1.7. Calpain 3 western blot

Protein extracts were prepared from muscle biopsies of the injected animals. Cryosections of the different muscles sampled were lysed in the following buffer Urea Buffer : 8M Urea, 2M Thiourea, 3% SDS, 50mM Tris-HCl pH 6.8, 0.03% Bromophenol Blue with EDTA-free Protease Inhibitors (cOmplete^{™} ULTRA Tablets, Mini EDTA-free, EASYpack Protease Inhibitor Cocktail Tablets). Quantification of protein concentration was performed using the Pierce BCA Protein Assay kit. After denaturation at 95°C for 10 min, 20 µg were loaded on a 4-12% Bis-Tris gel. After dry-transfer on nitrocellulose membrane, a Western blot was performed using antibodies against Calpain3 (Mouse antibody NCL-CALP-12A2, Novocastra, dilution 1:200 and Goat antibody COP-080049, Operon Biotechnologies). Membranes were incubated with primary antibodies overnight at 4C, after 3 washes in PBS, secondary antibodies were incubated 1h at room temperature. The secondary antibodies used were: anti-rabbit Donkey anti Rabbit 780, dilution 1/10000).

### 2. Results

The inventors have tested the capacity of P1 peptide (RGDLGLS (SEQ ID NO: 1); Michelfelder et al., PLoS ONE, 2009, 4, e5122) to increase the efficiency of transduction and transgene expression in the muscle after insertion in the AAV9-rh74 hybrid capsid (SEQ ID NO: 2) disclosed in WO2019/193119 as well as AAV9 and AAV8 capsid serotypes.

The P1-modified AAV9-rh74 hybrid capsid according to the invention was compared to P1-modified AAV9-rh74 hybrid capsid obtained according to the peptide insertion strategy into AAV capsid disclosed in Kienle EC (Dissertation, 2014; precited). Briefly, P1 sequence flanked by AAA and SG respectively in N-ter and C-ter was inserted at position 589 (e.g. between N589 and A590) of AAV9-rh74 hybrid capsid without modifying the capsid amino acid sequence upstream and downstream of the insertion site. In the P1-modified hybrid AAV9.rh74 capsid prepared according to Kienle EC, the ⁵⁸⁷QQN⁵⁸⁹ and ⁵⁹⁰AAP⁵⁹² sequences flanking the insertion site (respectively upstream and downstream of the insertion site) are changed to GQSG and AQAA, respectively, then peptide P1 is inserted in the hybrid AAV9.rh74 capsid. The resulting P1-modified hybrid capsid according to the present invention, named herein Cap9rh74-HB-P1 or AAV9-rh74-HB-P1 capsid, has the amino acid sequence SEQ ID NO : 6. The resulting P1-modified hybrid capsid according to Kienle EC, named herein Cap9rh74-K-P1 or AAV9-rh74-K-P1 capsid, has the amino acid sequence SEQ ID NO: 14. P1-modified hybrid AAV9.rh74 capsid (Cap9rh74-AKA-P1-AK) comprising P1 peptide sequence flanked by non-small amino acid sequences AKA and AK, respectively in N-ter and C-ter was tested as negative control. P1-modified AAV9 and AAV8 capsids according to the invention were also tested to assess the efficiency of the peptide P1 insertion strategy in other AAV serotypes.

To assess the biodistribution of the peptide-modified AAV capsids, recombinant AAV9-rh74, AAV9 and AAV8 vectors harboring the P1 peptide and GFP-Luciferase as transgene under the control of the CMV promoter were produced and injected (intravenously in 5 week-old B6 albino mice (4×10¹⁰ vg/mouse or 1×10¹¹ vg/mouse).

Acquisition of *in vivo* bioluminescence images was done 14 days post-injection, sacrifice and tissue sampling was done on day 21 after injection.

The *in vivo* bioluminescence images show that the addition of the P1 peptide in the AAV9.rh74 capsid using both the prior art strategy ("Strategy K") and the strategy of the invention ("Strategy HB") described before, leads in both cases to a higher luciferase expression in the posterior limbs.

However, the results of luciferase expression analysis in lysates of different tissues showed a lower signal in the liver of mice injected with AAV9-rh74-HB-P 1 capsid compared to the unmodified capsids and to AAV9-rh74-K-P1 **(****Figure 1** **and Table 1).** This demonstrates that AAV9-rh74-HB-P1 has an increased detargeting from the liver compared to AAV9-rh74-K-P1.

**Table 1: Decrease of liver targeting with AAV9-rh74-HB-P1 compared to AAV9-rh74-K-P1**

| | **Fold decrease** |
|---|---|
| **AAV9-rh74-HB-P1 vs AAV9** | 193,6 |
| **AAV9-rh74-K-P1 vs AAV9** | 9,9 |
| **AAV9-rh74-HB-P1 vs AAV9-rh74** | 88,6 |
| **AAV9-rh74-K-P1 vs AAV9-rh74** | 4,5 |

Vector copy number (VCN) analysis in the different organs of the injected mice shows that the P1 peptide insertion strategy according to the invention ("Strategy HB") improves muscle targeting, in particular in skeletal muscle and detargeting from the liver compared to prior art strategy ("Strategy K"), as assessed by a significantly higher (2 to 3 fold) skeletal muscle to liver ratio of vector copy number with AAV9-rh74-HB-P1 (AAV9rh74-AAA-P1-SG) compared to AAV9-rh74-K-P1 (AAV9rh74-GQSG-P1-AQAA) **(****Figure 3****).** By contrast, a low vector copy number in muscle and high vector copy number in liver resulting in a low muscle to liver ratio of vector copy number is observed with control vector comprising P1 modified AAV9.rh74 capsid flanked by non-small amino acid sequences (AAV9rh74-AKA-P1-AK; **Figure 3****).** This demonstrates the major contribution of the small-amino acid sequences flanking the P1 peptide in the improved tropism of the P1-modified AAV capsid according to the invention. Muscle targeting and detargeting from the liver were also observed with P1-modified AAV8 and AAV9 capsids according to the invention demonstrating that the peptide P1 insertion strategy according to the invention ("Strategy HB") is functional irrespective of the AAV serotype **(****Figure 4****).**

To further evaluate the efficiency of AAV9-rh74-HB-P1 compared to AAV9-rh74-K-P1 and to unmodified AAV9 in muscle, recombinant AAV vectors harboring the P1 peptide and calpain 3 as transgene under the control of a muscle specific promoter were produced and injected (intravenously in 1 month-old rat at the dose of 1 10e14 vg/kg (4×10¹⁰ vg/rat).

After sampling, muscles were subjected to Western blot. The result showed that the most efficient vector to transduce the muscle was AAV9-rh74-HB-P1 **(****Figure 2****).**

In conclusion, the inventors have identified a peptide-modified AAV capsid with a higher muscle transfer and liver detargeting, indicating that the mode of insertion of the peptide has an impact on the biodistribution of the AAV capsids.

## Claims

1. A modified adeno-associated virus (AAV) capsid protein comprising a muscle-targeting peptide insertion between two consecutive amino acids of the capsid protein sequence and without any modification of the capsid protein amino acid sequence upstream and downstream of the insertion site, wherein the targeting peptide comprises the sequence SEQ ID NO: 1 and a sequence of small amino-acids at the N-terminal and C-terminal ends of SEQ ID NO: 1, and wherein the insertion site is into the variable region IV, V or VIII of the AAV capsid protein sequence.

2. The modified AAV capsid protein of claim 1, wherein the sequence of small amino-acids consists of up to five amino acids selected from A, G, S, N; T, D, and L; preferably G, GST, AAA, SGS, SGA, AGA, GAA or ALA at the N-terminal end and G, SG, AA, TG, GG, SA, AG or GA at the C-terminal end; more preferably AAA at the N-terminal end and SG at the C-terminal end of SEQ ID NO: 1.

3. The modified AAV capsid protein of claim 1 or 2, wherein the muscle-targeting peptide consists of a sequence from 9 amino acids to up to 25, 20 or 15 amino acids.

4. The modified AAV capsid protein of any one of claims 1 to 3, wherein the insertion site is at a position selected from positions 587, 588, 589, 453, 520, 584 and 585, according to the numbering in AAV2 capsid protein sequence.

5. The modified AAV capsid protein of any one of claims 1 to 4, which is from an AAV serotype selected from the group consisting of: AAV6, AAV8, AAV9, AAVrh74 and AAV9.rh74; preferably AAV9 or AAV9.rh74.

6. The modified AAV capsid protein of claim 5, which is from AAV9.rh74 and comprises the muscle-targeting peptide insertion at position 589; preferably wherein the targeting peptide is selected from the group consisting of: SEQ ID NO: 5, 21, 23, 25, 27, 29, 31, 33, 35 and 37; more preferably SEQ ID NO: 5.

7. The modified AAV capsid protein of claim 6, which comprises a sequence selected from the group consisting of the sequence SEQ ID NO: 6 and the variant sequences having at least 85%, 87%, 88%, 90%, 95%, 97%, 98% or 99% identity with said sequence, wherein said variant sequences comprise no mutations in the muscle-targeting peptide sequence and the 5 amino acid sequence; preferably the 10 amino acid sequence before and after the insertion site.

8. The modified AAV capsid protein of any one of claims 1 to 7, which has an increased tropism for muscle, in particular heart and/or skeletal muscle, and/or a decreased tropism for liver.

9. A polynucleotide encoding the modified AAV capsid protein according to any one of claims 1 to 8; preferably comprising the sequence SEQ ID NO: 7 or a sequence having at least 80%, 85%, 90%, 95%, 97%, 98% or 99% identity with said sequence.

10. A recombinant plasmid comprising the polynucleotide of claim 9.

11. An AAV vector particle packaging a gene of interest, which comprises the modified AAV capsid protein according to any one of claims 1 to 8.

12. The AAV vector particle according to claim 11, wherein the gene of interest is selected from the group consisting of: therapeutic genes; genes encoding therapeutic proteins or peptides such as therapeutic antibodies or antibody fragments and genome editing enzymes; and genes encoding therapeutic RNAs such as interfering RNAs, guide RNAs for genome editing and antisense RNAs capable of exon skipping.

13. A pharmaceutical composition comprising a therapeutically effective amount of AAV vector particle according to claim 11 or 12, or cell stably transduced by said AAV vector particle of claim 11 or 12.

14. The pharmaceutical composition of claim 13, for use as a medicament in gene therapy, preferably for treating muscle diseases.

15. The pharmaceutical composition for use of claim 14, which targets a gene responsible for a muscle disease chosen from Dystrophinopathies and Limb-girdle muscular dystrophies; preferably a gene selected from the group comprising: *DMD, CAPN3, DYSF, FKRP, DNAJB6, ANO5, SGCA, SGCB* and *SGCG.*

## Patentansprüche

1. Modifiziertes Adeno-assoziiertes Virus-(AAV)-Kapsidprotein, umfassend eine Muskel-abzielende Peptidinsertion zwischen zwei aufeinanderfolgenden Aminosäuren der Kapsidproteinsequenz und ohne jegliche Modifikation der Kapsidprotein-Aminosäuresequenz stromaufwärts und stromabwärts der Insertionsstelle, wobei das abzielende Peptid die Sequenz SEQ ID NO: 1 und eine Sequenz von kleinen Aminosäuren an dem N-terminalen und C-terminalen Ende von SEQ ID NO: 1 umfasst, und wobei die Insertionsstelle in der variablen Region IV, V oder VIII der AAV-Kapsidproteinsequenz liegt.

2. Modifiziertes AAV-Kapsidprotein nach Anspruch 1, wobei die Sequenz von kleinen Aminosäuren aus bis zu fünf Aminosäuren besteht, die aus A, G, S, N; T, D und L ausgewählt sind; vorzugsweise G, GST, AAA, SGS, SGA, AGA, GAA oder ALA am N-terminalen Ende und G, SG, AA, TG, GG, SA, AG oder GA am C-terminalen Ende; stärker bevorzugt AAA am N-terminalen Ende und SG am C-terminalen Ende von SEQ ID NO: 1.

3. Modifiziertes AAV-Kapsidprotein nach Anspruch 1 oder 2, wobei das Muskel-abzielende Peptid aus einer Sequenz von 9 Aminosäuren bis zu 25, 20 oder 15 Aminosäuren besteht.

4. Modifiziertes AAV-Kapsidprotein nach einem der Ansprüche 1 bis 3, wobei sich die Insertionsstelle an einer Position befindet, die gemäß der Nummerierung in der AAV2-Kapsidproteinsequenz aus den Positionen 587, 588, 589, 453, 520, 584 und 585 ausgewählt ist.

5. Modifiziertes AAV-Kapsidprotein nach einem der Ansprüche 1 bis 4, das von einem AAV-Serotyp stammt, der ausgewählt ist aus der Gruppe bestehend aus: AAV6, AAV8, AAV9, AAVrh74 und AAV9.rh74; vorzugsweise AAV9 oder AAV9.rh74.

6. Modifiziertes AAV-Kapsidprotein nach Anspruch 5, das von AAV9.rh74 stammt und die Muskel-abzielende Peptidinsertion an Position 589 umfasst, vorzugsweise wobei das abzielende Peptid ausgewählt ist aus der Gruppe bestehend aus: SEQ ID NO: 5, 21, 23, 25, 27, 29, 31, 33, 35 und 37; stärker bevorzugt SEQ ID NO: 5.

7. Modifiziertes AAV-Kapsidprotein nach Anspruch 6, das eine Sequenz umfasst, die ausgewählt ist aus der Gruppe bestehend aus der Sequenz SEQ ID NO: 6 und den verschiedenen Sequenzen, die mindestens 85%, 87%, 88%, 90%, 95%, 97%, 98% oder 99% Identität mit dieser Sequenz aufweisen, wobei die verschiedenen Sequenzen keine Mutationen in der Muskel-abzielenden Peptidsequenz und der 5-Aminosäuresequenz umfassen; vorzugsweise die 10-Aminosäuresequenz vor und nach der Insertionsstelle.

8. Modifiziertes AAV-Kapsidprotein nach einem der Ansprüche 1 bis 7, das einen erhöhten Tropismus für Muskeln, insbesondere Herz- und/oder Skelettmuskeln, und/oder einen verringerten Tropismus für Leber aufweist.

9. Polynukleotid, das für das modifizierte AAV-Kapsidprotein nach einem der Ansprüche 1 bis 8 kodiert; vorzugsweise umfassend die Sequenz SEQ ID NO 7 oder eine Sequenz, die mindestens 80%, 85%, 90%, 95%, 97%, 98% oder 99% Identität mit dieser Sequenz aufweist.

10. Rekombinantes Plasmid, umfassend das Polynukleotid nach Anspruch 9.

11. AAV-Vektorpartikel, das ein Gen von Interesse verpackt, das das modifizierte AAV-Kapsidprotein nach einem der Ansprüche 1 bis 8 umfasst.

12. AAV-Vektorpartikel nach Anspruch 11, wobei das Gen von Interesse ausgewählt ist aus der Gruppe bestehend aus: therapeutischen Genen; Genen, die für therapeutische Proteine oder Peptide kodieren, wie z.B. therapeutische Antikörper oder Antikörperfragmente und Genom-Editierungsenzyme; und Genen, die für therapeutische RNAs kodieren, wie z.B. interferierende RNAs, Guide-RNAs für ein Genom-Editieren und Antisense-RNAs, die für ein Überspringen von Exons geeignet sind.

13. Pharmazeutische Zusammensetzung, umfassend eine therapeutisch wirksame Menge eines AAV-Vektorpartikels nach Anspruch 11 oder 12, oder eine Zelle, die durch das AAV-Vektorpartikel nach Anspruch 11 oder 12 stabil transduziert ist.

14. Pharmazeutische Zusammensetzung nach Anspruch 13 zur Verwendung als ein Medikament in einer Gentherapie, vorzugsweise zur Behandlung von Muskelkrankheiten.

15. Pharmazeutische Zusammensetzung zur Verwendung nach Anspruch 14, die auf ein Gen abzielt, das für eine Muskelkrankheit verantwortlich ist, die aus Dystrophinopathien und Gliedergürtel-Muskeldystrophien gewählt ist, vorzugsweise ein Gen, ausgewählt aus der Gruppe, umfassend: DMD, CAPN3, DYSF, FKRP, DNAJB6, AN05, SGCA, SGCB und SGCG.

## Revendications

1. Protéine de capside de virus adéno-associé (AAV) modifiée comprenant une insertion de peptide de ciblage des muscles entre deux acides aminés consécutifs de la séquence de protéine de capside et sans aucune modification de la séquence d'acides aminés de la protéine de capside en amont et en aval du site d'insertion, dans laquelle le peptide de ciblage comprend la séquence SEQ ID NO : 1 et une séquence de petits acides aminés aux extrémités N-terminale et C-terminale de SEQ ID NO : 1, et dans laquelle le site d'insertion se trouve dans la région variable IV, V ou VIII de la séquence de protéine de capside AAV.

2. Protéine de capside AAV modifiée selon la revendication 1, dans laquelle la séquence de petits acides aminés est constituée de cinq acides aminés au maximum sélectionnés parmi A, G, S, N ; T, D et L ; de préférence G, GST, AAA, SGS, SGA, AGA, GAA ou ALA à l'extrémité N-terminale et G, SG, AA, TG, GG, SA, AG ou GA à l'extrémité C-terminale ; de manière d'avantage préférée AAA à l'extrémité N-terminale et SG à l'extrémité C-terminale de SEQ ID NO : 1.

3. Protéine de capside AAV modifiée selon la revendication 1 ou 2, dans laquelle le peptide de ciblage des muscles est constitué d'une séquence de 9 acides aminés jusqu'à 25, 20 ou 15 acides aminés.

4. Protéine de capside AVV modifiée selon l'une quelconque des revendications 1 à 3, dans laquelle le site d'insertion est à une position sélectionnée parmi les positions 587, 588, 589, 453, 520, 584 et 585, selon la numérotation dans la séquence de protéine de capside d'AAV2.

5. Protéine de capside AAV modifiée selon l'une quelconque des revendications 1 à 4, qui provient d'un sérotype d'AAV sélectionné dans le groupe constitué de : AAV6, AAV8, AAV9, AAVrh74 et AAV9.rh74 ; de préférence AAV9 ou AAV9.rh74.

6. Protéine de capside AAV modifiée selon la revendication 5, qui provient d'AAV9.rh74 et comprend l'insertion du peptide de ciblage des muscles en position 589 ; de préférence, dans lequel le peptide de ciblage est sélectionné dans le groupe constitué de : SEQ ID NO : 5, 21, 23, 25, 27, 29, 31, 33, 35 et 37 ; de manière d'avantage préférée SEQ ID NO : 5.

7. Protéine de capside AAV modifiée selon la revendication 6, qui comprend une séquence sélectionnée dans le groupe constitué de la séquence SEQ ID NO : 6 et des séquences variantes présentant au moins 85 %, 87 %, 88 %, 90 %, 95 %, 97 %, 98 % ou 99 % d'identité avec ladite séquence, dans laquelle lesdites séquences variantes ne comprennent pas de mutations dans la séquence du peptide de ciblage des muscles et la séquence de 5 acides aminés ; de préférence la séquence de 10 acides aminés avant et après le site d'insertion.

8. Protéine de capside AAV modifiée selon l'une quelconque des revendications 1 à 7, qui présente un tropisme accru pour le muscle, en particulier le muscle cardiaque et/ou squelettique, et/ou un tropisme diminué pour le foie.

9. Polynucléotide codant pour la protéine de capside d'AAV modifiée selon l'une quelconque des revendications 1 à 8 ; comprenant de préférence la séquence SEQ ID NO : 7 ou une séquence présentant au moins 80 %, 85 %, 90 %, 95 %, 97 %, 98 % ou 99 % d'identité avec ladite séquence.

10. Plasmide recombiné comprenant le polynucléotide selon la revendication 9.

11. Particule de vecteur AAV encapsidant un gène d'intérêt, qui comprend la protéine de capside AAV modifiée selon l'une quelconque des revendications 1 à 8.

12. Particule de vecteur AAV selon la revendication 11, dans laquelle le gène d'intérêt est sélectionné dans le groupe constitué : des gènes thérapeutiques ; des gènes codant pour des protéines ou peptides thérapeutiques tels que des anticorps ou fragments d'anticorps thérapeutiques et des enzymes d'édition du génome ; et des gènes codant pour des ARN thérapeutiques tels que des ARN interférents, des ARN guides pour l'édition du génome et des ARN antisens capables de sauter des exons.

13. Composition pharmaceutique comprenant une quantité thérapeutiquement efficace de particule de vecteur AAV selon la revendication 11 ou 12, ou une cellule transduite de manière stable par ladite particule de vecteur AAV selon la revendication 11 ou 12.

14. Composition pharmaceutique selon la revendication 13, pour une utilisation comme médicament en thérapie génique, de préférence pour traiter les maladies musculaires.

15. Composition pharmaceutique pour une utilisation selon la revendication 14, qui cible un gène responsable d'une maladie musculaire choisie parmi les dystrophinopathies et les dystrophies musculaires scapulo-humérales ; de préférence un gène sélectionné dans le groupe comprenant : *DMD, CAPN3, DYSF, FKRP, DNAJB6, ANO5, SGCA, SGCB* et *SGCG.*
